# EUROPÄISCHE PATENTSCHRIFT

(11) **EP 2 086 976 B1**
(45) Veröffentlichungstag und Bekanntmachung des Hinweises auf die Patenterteilung: **18.05.2011**
(21) Anmeldenummer: 07818763.0
(22) Anmeldetag: 06.10.2007
(51) Int. Cl.: C07D 471/04

(54) **VERFAHREN ZUR HERSTELLUNG VON PRADOFLOXACIN**
METHOD FOR PRODUCING PRADOFLOXACIN
PROCÉDÉ DE PRODUCTION DE PRADOFLOXACINE

(30) Priorität: 20.10.2006 DE 102006049520
(43) Veröffentlichungstag der Anmeldung: 12.08.2009
(73) Patentinhaber: Bayer Animal Health GmbH, 51368 Leverkusen (DE)
(72) Erfinder: ADAM, Thomas, 42105 Wuppertal (DE); WISCHNAT, Ralf, 51467 Bergisch Gladbach (DE); WEIDEMANN, Klaus, 42279 Wuppertal (DE)
(86) Internationale Anmeldenummer: PCT/EP2007/008687
(87) Internationale Veröffentlichungsnummer: WO 2008/046532

(56) Entgegenhaltungen:
- DE-A1- 19 908 449

## Beschreibung

Die Erfindung betrifft ein verbessertes Verfahren zur Herstellung von Pradofloxacin, bei dem der Substituent in 7-Position durch nukleophile Substitution in einem N-Methylpyrrolidon-Ethanol-Lösungsmittelgemisch eingeführt wird.

Das Chinolon-Antibiotikum Pradofloxacin der Formel (I) 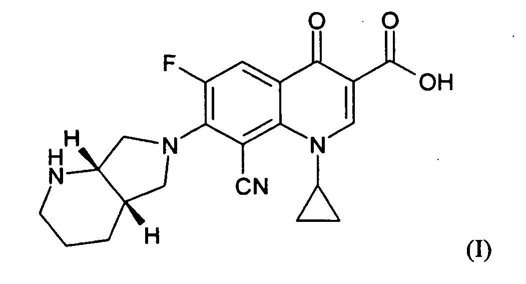 und seine Herstellung sind bereits in WO 97/31001 beschrieben. Bestimmte Vorstufen, Zwischenprodukte und frühe Verfahrensschritte werden in WO98/47862 und WO99/06360 offenbart.

Schlüsselschritt der Herstellung ist die Umsetzung der entsprechenden 7-Halogen-Cyanofluorchinolonsäure, insbesondere der 7-Chlor-8-cyan-1-cyclopropyl-6-fluor-1,4-dihydro-4-oxo-3-chinolincarbonsäure der Formel (II) 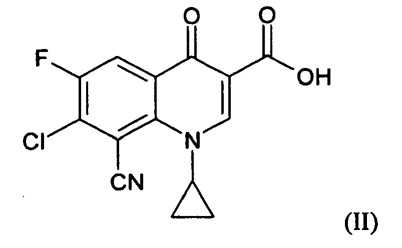 mit (1S,6S)-2,8-Diazabicyclo-[4.3.0]nonan der Formel (III) (auch als S,S-Pyrrolopiperidin bezeichnet) 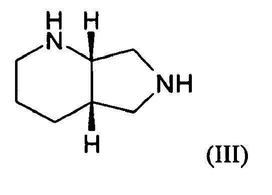 in einer nukleophilen Substitutionsreaktion. Bekannt ist, dass solche nukleophilen Substitutionen vorzugsweise in polar aprotischen Lösungsmittlen durchgeführt werden. So schlägt WO 97/31001 in allgemeiner Form unter anderem Dimethylsulfoxid, NN-Dimethylformamid, N-Methylpyrrolidon, Hexamethylphosphorsäuretriamid, Sulfolan und Acetonitril für entsprechende Umsetzungen vor; Alkohole wie z. B. Methanol, Ethanol, n-Propanol, Isopropanel und andere werden dort ebenfalls als Lösungsmittel für diese Zwecke vorgeschlagen. DE 19808449 Beschreibt ein Verfahren in einem Lösungsmittelgemisch aus ethanol N-Methylpyrrolidon im Verhältnis 60:40.

Überraschenderweise wurde festgestellt, dass als Lösungsmittel für diese Reaktion ein Gemisch von N-Methylpyrrolidon mit Ethanol besonders geeignet ist.

Die Erfindung betrifft ein Verfahren zur Herstellung von Pradofloxacin der Formol (I) 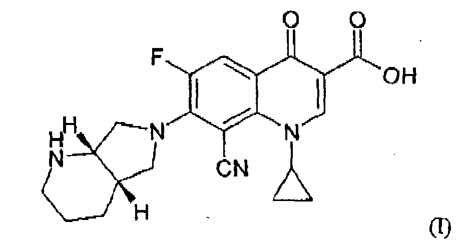 durch Umsetzung, gegebenenfails in Anwesenheit einer Base, der 7-Chlor-8-cyan-1-cyzlopropyl-6-fluor-1,4-dihydro-4-oxo-3-chinolincarbonsäure der Formel (II) 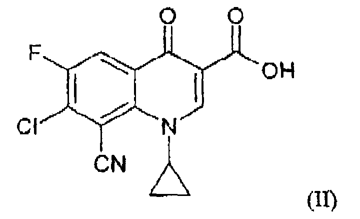 mit (1S,6S)-2,8-Diazabicyclo-[4.3.0]nonan der Formel (III) 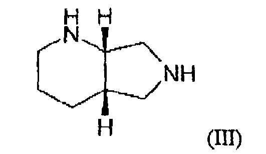 in einem Lösungsmittelgemisch aus N-Methylpyrrolidon und Ethanol, wobei das Lösungsmittelgemisch 70-90 Gew.% Ethanol enthält.

Für das Verhältnis von (1S,6S)-2,8-Diazabicyclo-[4.3.0]nonan zu 7-Chlor-8-cyan-1-cyclopropyl-6-fluor-1,4-dihydro-4-oxo-3-chinolincarbonsäure wird in WO 97/31001 ein weiter Bereich angegeben, der von äquimolaren Mengen bis zu einem großen Überschuss an (1S,6S)-2,8-diazabicyclo-[4.3.0]nonan reicht. Bei der Entwicklung einer in technischem Maßstab durchführbaren Synthese wird der Fachmann routinemäßig einen Überschuss an (1S,6S)-2,8-diazabicyclo-[4.3.0]nonan wählen: (1S,6S)-2,8-Diazabicyclo-[4.3.0]nonan hat zwei basische Ringstickstoffatome, die beide nukleophil mit der 7-Chlor-8-cyan-1-cyclopropyl-6-fluor-1,4-dihydro-4-oxo-3-chinolincarbonsäure reagieren können. Bei einer zu geringen Menge an (1S,6S)-2,8-Diazabicyclo-[4.3.0]nonan würde der Fachmann also die Bildung unerwünschter Nebenprodukte durch Verknüpfung des zweiten Ringstickstoffs des (1S,6S)-2,8-Diazabicyclo-[4.3.0]nonans mit einem weiteren Molekül 7-Chlor-8-cyan-1-cyclopropyl-6-fluor-1,4-dihydro-4-oxo-3-chinolincarbonsäure erwarten.

Unerwarteterweise hat es sich als günstig erwiesen, das (1S,6S)-2,8-Diazabicyclo-[4.3.0]nonan im Verhältnis zur 7-Chlor-8-cyan-1-cyclopropyl-6-fluor-1,4-dihydro-4-oxo-3-chinolincarbonsäure nur in einem geringen molaren Überschuss einzusetzen, und zwar üblicherweise die 1,01- bis 1,30-fache, bevorzugt die 1,05- bis 1,25-fache molare Menge bezogen auf die Menge an 7-Chlor-8-cyan-1-cyclopropyl-6-fluor-1,4-dihydro-4-oxo-3-chinolincarbonsäure.

Die Umsetzung von (1S,6S)-2,8-Diazabicyclo-[4.3.0]nonan mit 7-Chlor-8-cyan-1-cyclopropyl-6-fluor-1,4-dihydro-4-oxo-3-chinolincarbonsäure erfolgt in Gegenwart einer Base, welche die entstehende Säure bindet. Als solche kann z. B. ein Überschuss an (1S,6S)-2,8-Diazabicyclo-[4.3.0]nonan dienen, was aus den oben genannten Gründen weniger bevorzugt ist. Eingesetzt werden können generell anorganische und organische Basen. Hierzu gehören beispielsweise die Alkalihydroxide, Alkalicarbonate, organische Amine und Amidine. Bevorzugt sind organische Basen, insbesondere tertiäre Amine. Als besonders geeignet seien im Einzelnen genannt: 1,4-Diazabicyclo[2.2.2]octan (DABCO), 1,8- Diazabicyclo[5.4.0]undec-7-en (DBU), Triethylamin, Tributylamin und insbesondere Diisopropylethylamin.

Die Reaktion kann in einem breiten Temperaturbereich von 0 bis 200°C, bevorzugt 20 bis 180°C durchgeführt werden. Die Umsetzung erfolgt üblicherweise bei Normaldruck, eine Durchführung bei erhöhtem Druck z. B. bei 1 bis 100 bar, bevorzugt 1 bis 10 bar ist jedoch denkbar.

Unter den erfindungsgemäßen Bedingungen lässt sich die Reaktion reproduzierbar mit guter Ausbeute durchführen, das Pradofloxacin wird in sehr guter Reinheit erhalten, der Aufwand für die Reinigung des Endproduktes kann deutlich reduziert werden, in der Regel sind weitere Reinigungsschritte nicht mehr erforderlich. Insbesondere bei der Herstellung im technischen Maßstab sind diese Vorteile von großer Bedeutung.

Pradofloxacin ist ein hoch wirksames neues Chinolonantibiotikum, seine antibakterielle Wirkung sowie Indikationen, Anwendungsformen und geeignete Zubereitungen sind im Stand der Technik bereits beschrieben, siehe z. B. WO 97/31001, WO 03/007995, WO 03/101422, WO 04/082658, WO 05/018641, WO 05/044271 und WO 06/061156.

### Beispiel

100 g 7-Chlor-8-eyan-l-cyclopropyl-6-fluor-1,4-dihydro-4-oxo-3-chinolincarbonsäure werden in einer Mischung aus Ethanol und N-Methylpyrrolidon (80/20 w/w) unter Zusatz eines Überschusses an Diisopropylethylamin bei erhöhter Temperatur (>70 °C) mit 48 g (1S,6S)-2,8-Diazabicyclo-[4.3.0]nonan umgesetzt. Es wird nach Abkühlen eine Ausbeute von 90 % d.Th. erhalten.

## Patentansprüche

1. Verfahren zur Herstellung von Pradofloxacin der Formel (I) 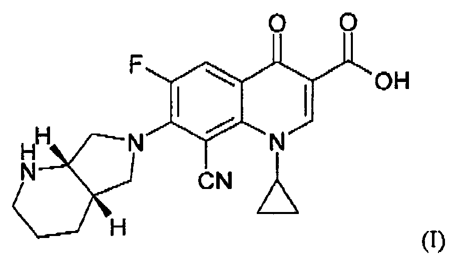 durch Umsetzung, gegebenenfalls in Anwesenheit einer Base, der Verbindung der Formel (II) 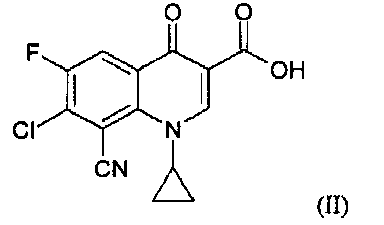 mit (1S,6S)-2,8-Diazabicyclo-[4.3.0]nonan der Formel (III) 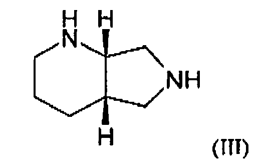 in einem Lösungsmittelgemisch aus N-Methylpyrrolidon und Ethanol, wobei das Lösungsmittelgemisch 70 bis 90 Gew.-% Ethanol enhält

2. Verfahren gemäß Anspruch 1, wobei das (1S,6S)-2,8-Diazabicyclo-[4.3.0]nonan der Formel (III) in einem molaren Verhältnis zur 7-Chlor-8-cyan-1-cyclopropyl-6-fluor-1,4-dihydro-4-oxo-3-chinolincarbonsäure der Formel (II) von 1 : 1,01 bis 1 : 1,30 eingesetzt wird.

## Claims

1. Process for preparing pradofloxacin of the formula (I) 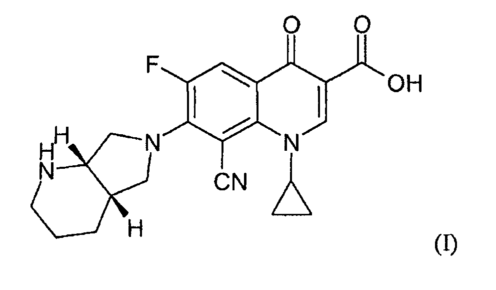 by reacting, optionally in the presence of a base, the compound of the formula (II) 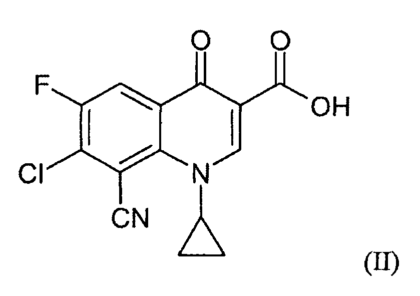 with (1S,6S)-2,8-diazabicyclo-[4.3.0]nonane of the formula (III) 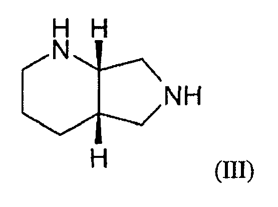 in a solvent mixture of N-methylpyrrolidone and ethanol, wherein the solvent mixture contains 70 to 90% by weight of ethanol.

2. Process according to Claim 1, wherein the (1S,6S)-2,8-diazabicyclo[4.3.0]nonane of the formula (III) is used in a molar ratio to the 7-chloro-8-cyano-1-cyclopropyl-6-fluoro-1,4-dihydro-4-oxo-3-quinolinecarboxylic acid of the formula (II) of 1:1.01 to 1:1.30.

## Revendications

1. Procédé de préparation de pradofloxacine de formule (I) 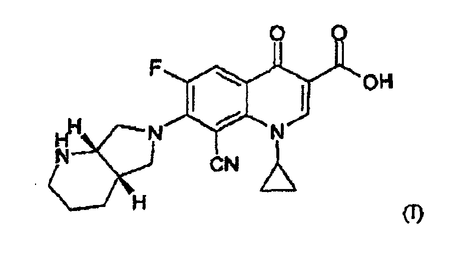 par réaction, éventuellement en présence d'une base, du composé de formule (II) 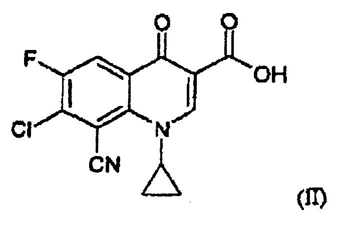 avec du (1S,6S)-2,8-diazabicyclo[4.3.0]nonane de formule (III) 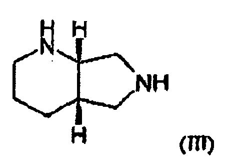 dans un mélange de solvants constitué de N-méthylpyrrolidone et d'éthanol, le mélange de solvants contenant 70 à 90 % en poids d'éthanol.

2. Procédé selon la revendication 1, dans lequel le (1S,6S)-2,8-diazabicyclo[4.3.0]nonane de formule (III) est utilisé selon un rapport en moles avec l'acide 7-chloro-8-cyano-1-cyclopropyl-6-fluoro-1,4-dihydro-4-oxo-3-quinoléinecarboxylique de formule (II) de 1:1,01 à 1:1,30.
